# EUROPEAN PATENT SPECIFICATION

(11) **EP 1 328 291 B1**
(45) Date of publication and mention of the grant of the patent: **24.12.2008**
(21) Application number: 01975472.0
(22) Date of filing: 26.09.2001
(51) Int. Cl.: A61K 39/235, C12N 15/09, C12N 15/19, C12N 15/33, C12N 15/34, C12N 15/64, C12N 15/85, C12N 15/86, C12N 15/861, A61K 48/00

(54) **VIRUSES TARGETED TO HYPOXIC CELLS AND TISSUES**
VIREN GEGEN HYPOXISCHE ZELLEN UND GEWEBE
VIRUS DIRIGES SUR DES CELLULES ET DES TISSUS HYPOXIQUES

(30) Priority: 26.09.2000 US 235283 P
(43) Date of publication of application: 23.07.2003
(73) Proprietor: EMORY UNIVERSITY, Atlanta, GA 30322 (US)
(72) Inventor: VAN MEIR, Erwin, Tucker, GA 30084 (US); NICHOLSON, Ainsley, C., Tucker, GA 30084 (US); POST, Dawn, E., Duluth, GA 30096 (US)
(74) Representative: Fisher, Adrian John
(86) International application number: PCT/US2001/030236
(87) International publication number: WO 2002/026192

(56) References cited:
- WO-A-00/15820
- WO-A-98/05797
- WO-A2-99/23216
- DACHS G U ET AL: "Targeting gene expression to hypoxic tumor cells" NATURE MEDICINE, NATURE PUBLISHING, CO, US, vol. 3, no. 5, May 1997 (1997-05), pages 515-520, XP002096343 ISSN: 1078-8956
- RUAN H ET AL: "A HYPOXIA-REGULATED ADENO-ASSOCIATED VIRUS VECTOR FOR CANCER-SPECIFIC GENE THERAPY" NEOPLASIA, DOYMA, BARCELONA,, ES, vol. 3, no. 3, 2001, pages 255-263, XP002909242 ISSN: 0212-9787
- RODRIGUEZ R ET AL: "PROSTATE ATTENUATED REPLICATION COMPETENT ADENOVIRUS (ARCA) CN706: A SELECTIVE CYTOTOXIC FOR PROSTATE-SPECIFIC ANTIGEN-POSITIVE PROSTATE CANCER CELLS" CANCER RESEARCH, AMERICAN ASSOCIATION FOR CANCER RESEARCH, BALTIMORE, MD, US, vol. 57, 1 July 1997 (1997-07-01), pages 2559-2563, XP000982824 ISSN: 0008-5472
- TANAKA T ET AL: "VIRAL VECTOR-TARGETED ANTIANGIOGENIC GENE THERAPY UTILIZING AN ANGIOSTATIN COMPLEMENTARY DNA" CANCER RESEARCH, AMERICAN ASSOCIATION FOR CANCER RESEARCH, BALTIMORE, MD, US, vol. 58, no. 15, 1 August 1998 (1998-08-01), pages 3362-3369, XP000857409 ISSN: 0008-5472
- BARON U ET AL: "CO-REGULATION OF TWO GENE ACTIVITIES BY TETRACYCLINE VIA A BIDIRECTIONAL PROMOTER" NUCLEIC ACIDS RESEARCH, OXFORD UNIVERSITY PRESS, SURREY, GB, vol. 23, no. 17, 11 September 1995 (1995-09-11), pages 3605-3606, XP000775822 ISSN: 0305-1048
- POST D E ET AL: "A novel hypoxia-inducible factor (HIV) activated oncolytic adenovirus for cancer therapy" ONCOGENE, BASINGSTOKE, HANTS, GB, vol. 22, no. 14, 10 March 2003 (2003-03-10), pages 2065-2072, XP002975912 ISSN: 0950-9232
- RUAN H. ET AL.: 'A hypoxia-regulated adeno-associated virus vector for cancer-specific gene therapy' NEOPLASIA vol. 3, no. 3, May 2001 - June 2001, pages 255 - 263, XP002909242
- RUAN H. ET AL.: 'Use of hypoxia-regulated gene expression in tumor-specific gene therapy' CURR. OPIN. INVESTIGATIONAL DRUGS vol. 2, no. 6, June 2001, pages 839 - 843, XP002909241
- IDO A. ET AL.: 'Gene therapy targeting for hepatocellular carcinoma: selective and enhanced suicide gene expression regulated by a hypoxia-inducible enhancer linked to a human alpha fetoprotein promotor' CANCER RESEARCH vol. 61, no. 7, April 2001, pages 3016 - 3021, XP002909240
- CHO ET AL.: "Oncolytic effects of adenovirus mutant capable of replicating in hypoxic and normoxic regions of solid tumor" vol. 10, no. 5, November 2004 (2004-11),

### SEQUENCE LISTING

<110> EMORY UNIVERSITY
   <110> POST, Dawn
   <110> NICHOLSON, Ainsley
   <110> VAN MEIR, Erwin
<120> VIRUSES TARGETED TO HYPOXIC CELLS AND TISSUES
<130> P033595EP
<140> 01975472.0
   <141> 2001-09-26
<150> 60/235283
   <151> 2000-09-26
<160> 2
<170> SeqWin99, version 1.02
<210> 1
   <211> 33
   <212> DNA
   <213> Artificial Sequence
<220>
   <223> EPO HRE sequence
<400> 1
   gccctacgtg ctgtctcaca cagcctgtct gac 33
<210> 2
   <211> 35
   <212> DNA
   <213> Artificial Sequence
<220>
   <223> VEGF sequence
<400> 2
   ccacagtgca tacgtgggct ccaacaggtc ctctt 35

## Description

### ACKNOWLEDGMENT OF GOVERNMENT FUNDING

This invention was made, at least in part, with funding from the National Institutes of Health under grant no. NS41403.

### FIELD OF THE INVENTION

This invention generally relates to a novel recombinant viruses that selectively targets cells in which the partial oxygen pressure is less than normally found in that particular tissue type or which have an activated HIF (Hypoxia-Inducible Factor)pathway. The invention further relates to a virus that selectively replicates and is cytolytic in cells and tissues that are hypoxic or have an activated HIF pathway. Methods of treating cells or tissues that are hypoxic with the novel compositions of the invention are also provided. The invention further relates to screening assays for identifying compounds that modulate the hypoxia inducible pathway.

### BACKGROUND OF THE INVENTION

Cancer is one of the leading causes of death in industrialized countries. In the United States, cancer is the second leading cause of all deaths and accounts for hundreds of thousands of deaths each year. Cancer is a devastating disease on many levels. For example, gliomas are the main cause of death of patients with brain tumors. Patients with glioblastoma have a mean survival time of less than 12 months, and this prognosis has not changed much since 1959 (ten months) and 1932 (six to nine months) despite impressive developments in methods for treating cancer.

Cancer may be treated by a variety of methods including surgery, radiotherapy, chemotherapy, and immunotherapy. Although these methods of treatment have in general improved the survival rates of cancer victims, the fact remains that there is a clear need for improved therapeutic techniques for combating cancer.

A resurgent approach to treating cancer is referred to as virotherapy. Virotherapy first garnered the interest of scientists when it was discovered that tumors of some cancer patients regressed after they experienced viral infection or vaccination (see the reviews of Sinkovics, J., & Horvarth, J. [1993] Intervirology 36:193-214 and Alemany et al. [2000], Nat. Biotech. 18:723-727). Unfortunately, the initial promise of this approach was diminished when researchers discovered toxicity problems associated with virotherapy and furthermore the treatments had limited efficacity.

The advent of modem molecular biology has prompted scientists to reassess the feasibility of virotherapy. In particular, virus mediated gene therapy is now the central focus in the renewed interest in virotherapy. The molecular strategy underlying the design of virus mediated gene therapy systems is to deliver a gene which will inhibit tumor cell growth (*e.g.,* controlling cell cycle or apoptosis), kill the cell (suicide gene), or induce an immune response (immunotherapy).

Two general approaches are available. One approach has centered on the use of replication-deficient viral vectors. The use of replication deficient vectors as virotherapeutic agents has encountered two major problems: (1) low *in vivo* transduction efficiency, resulting in poor gene delivery and (2) inability to specifically target tumor versus normal tissue.

Another approach to virotherapy involves the use of replication-competent viruses. The use of replication-competent viruses with a cytolytic cycle, has emerged as a viable strategy for directly killing tumor cells (oncolysis) as well as enhancing gene transfer and specifically targeting tumor cells. A variety of modified neuroattenuated herpes simplex viruses, (HSVs) with deletions in the genes for neurovirulence (γ₁34.5) and ribonucleotide reductase (U_{L}39), function as oncolytic agents for human tumor cells *in vitro* and in mouse models of human brain tumors *in vivo.* See, *e.g*., Parker et al. 2000 PNAS 97:2208-2213; U.S. Patent 5,728,379). In phase 1 clinical trials, twenty-one patients with malignant glioma have received intracranial injections of HSV G207 without any signs of encephalitis or CNS changes (Markert et al. 2000 Gene Ther. 10:867-874).

A different strategy makes use of an oncolytic, replication competent adenovirus (dl1520/ONYX-015) which has a deletion that leads to abrogated production of the 55kDa E1B protein (Bischoff et al. 1996 Science 274:373-376) (See, *e.g.,* U.S. patents 6,080,578 and 5,677,178) Preliminary data suggested that the replication of this virus was restricted to tumor cells with a deficient p53 tumor suppressor gene. However, more recent findings have established that cells which are wild type for p53 gene status can also support replication of this virus (Rothmann et al. 1998 J. Virol. 72:9470-9478; Goodrun & Ornelles 1998 J. Virol. 72:9479-9490). This virus is currently being used in phase 1 clinical trials for ovarian cancer and gastrointestinal cancers that have metastasized to the liver, as well as in phase 2 and 3 clinical trials for recurrent and refractory head & neck cancer. Results so far have shown that injection of replication-competent adenovirus d11520 is safe and well-tolerated by patients, whose complaints are mainly minor grade 1-2 flu-like symptoms. (Kim [2000] Oncogene 19:6660-6669.) The low toxicity of these two viral systems in humans suggests that replication-competent viruses are promising approaches for treating patients with tumors. More recently, the design of oncolytic viruses whose replication is restricted to a specific tumor type has been realized. An adenovirus (CN706) was created which showed a selective cytotoxicity for prostate-specific antigen (PSA) positive cancer cells *in vitro* and in murine prostate cancer models *in vivo* (Rodriguez et al. 1997 Cancer Res. 57:2559-2563). (See, *e.g*., U.S. patents 5,871,726 and 6,197,293 .)

While such approaches utilizing replication-competent viruses are promising, they are limited in that: (1) multiple viruses would have to be created for different tumor types and possibly individual tumors due to the genetic heterogeneity of tumors, (2) they do not provide for the selective targeting of tumors derived from a broad range of tissues, and (3) they potentially require rigorous anti-viral treatments to eliminate virus after completion of therapy.

A multitude of U.S. patents have issued regarding hypoxia-inducible factor-1, virus mediated gene delivery, tissue specific constructs, and related topics.

Several patents to Semenza and Semenza *et al.* relate to hypoxia-inducible factor-1. U.S. patent 6,222,018 to Semenza discloses a substantially purified hypoxia-inducible factor (HIF-1) characterized as being capable of activating gene expression in genes that contain an HIF-1 binding site. U.S. patent 6,124,131 to Semenza discloses a substantially purified stable human hypoxia-inducible factor-1α as well as nucleotides encoding the same. U.S. patent 5,882,914 to Semenza discloses nucleic acids encoding hypoxia inducible factor-1 as well as purification and characterization of the expressed proteins.

The patents to Semenza and Semenza *et al.* refer to purified HIF-1, nucleic acids encoding HIF-1, antibodies that bind HIF-1, mutants of HIF-1, and method of using all of these biological molecules. The patents do not describe recombinant viruses that selectively replicate in and cytolyse hypoxic tissue and have the capability of delivering an anti-angiogenic factor.

U.S. patent 6,218,179 to Webster et al*.* discloses tissue-specific hypoxia regulated constructs. Webster *et al.* describe a method for reducing ischemic injury to a cell exposed to hypoxic conditions. The constructs for reducing ischemic injury described in Webster *et al.* comprise a chimeric gene containing an hypoxia responsive element, a therapeutic gene and a tissue-specific promoter. The therapeutic gene is selected so that its expression is effective in reducing ischemic injury to the cell. Examples of therapeutic genes are thos for nitric oxide synthase, Bcl-2, superoxide dismutase and catalase. U.S. patent 5,834,306 to Webster et al*.* discloses a method and compositions comprising chimeric genes. The chimeric genes contain a tissue-specific promoter and an hypoxia responsive enhancer element, both of which are operably linked to a selected gene.

Recombinant adeno-associated virions and methods of using them are described in a series of patents to Podsakoff *et al*. U.S. patent 6,211,163 to Podsakoff et al*.* discloses methods for delivering DNA to the bloodstream using recombinant adeno-associated virus vectors. The invention is based on the discovery that recombinant adeno-associated virions are efficiently delivered to various muscle cell types and provide for the sustained production of therapeutic proteins. U.S. patent 5,858,351 to Podsakoff *et al.* discloses the use of adeno-associated virus virions for delivering DNA molecules to muscle cells and tissues. U.S. patent 5,846,528 to Podsakoff et al*.* discloses recombinant adeno-associated virus virions for delivering DNA molecules to muscle cells and tissues in the treatment of anemia. The Podsakoff *et al.* patents do not describe recombinant viruses which oncylyse hypoxic tissue and provide an anti-angiogenic factor.

U.S. patents related to the hypoxia-inducible factor-1 pathway describe a number of strategies. U.S. patent 6,184,035 to Csete et al*.* discloses methods for isolating, activating, and controlling differentiation from skeletal muscle stem or progenitor cells by using hypoxic conditions. The patent to Csete *et al.* relates to the discovery that adult skeletal muscle fibers cultured under hypoxic conditions give rise to greater numbers of progenitor cells as compared to muscle fibers grown under normal oxygen levels. U.S. patent 6,130,071 to Alitalo et al*.* discloses purified and isolated vascular endothelial growth factor-C cysteine deletion variants. U.S. patent 5,952,226 to Aebischer et al. discloses a device and method for delivery of EPO to a patient using an implanted device that continuously releases EPO. The invention described in the Aebischer *et al*. patent relates to providing EPO to a subject with cells engineered to express high levels of EPO under hypoxic conditions. U.S. patent 5,681,706 to Anderson et al*.* discloses genetic regulatory elements which effect anoxic induction of a DNA molecule in mammalian cells exposed to anoxia. U.S. patent 5,942,434 to Ratcliffe et al*.* discloses nucleic acid constructs comprising hypoxia response elements operably linked with a coding sequence such as genes for pro-drug activation systems or cytokines. As seen from these patents, the hypoxia-inducible pathway was harnessed for use in a some specific contexts, but as far as the Applicant is aware, recombinant viruses which selectively replicate in and cytolyse hypoxic tissue, or tissues with and activated HIF pathway, and further deliver adjuvant threapy, are not described in the prior art

Thus, there is a need for virotherapeutic systems which combine a therapeutic gene delivery approach and an oncolytic mechanism for the selective targeting of a wide variety of tumors.

A variety of particular problems were encountered during the discovery of this invention. Infection of cells by viruses is a complicated biochemical process. It was first necessary to show that tumor cells could be infected by a recombinant adenovirus. Next, hypoxia-induced expression of constructs in transfected tumor cell lines had to be demonstrated. The HIF-activated expression also had to have an appropriate O₂ concentration versus expression level profile. Hypoxia inducible constructs that bi-directionally expressed gene products had to be designed and tested for embodiments of the invention that involve delivery of adjuvant therapy. After these initial stages of design and testing, recombinant viruses which contained the constructs were examined in transfected tumor cell lines for expression of E1A and E1B gene products. These expression studies demonstrated that hypoxia-dependent regulation seen in the transient reporter gene assay was maintained in the context of the viral genome. The next step in the invention involved demonstrating that the recombinant virus cytolyzed tumor cells in an hypoxia-dependent manner. Lastly, the inventors showed that the recombinant virus was delivered to brain tumors in a model system. Finally, these studies led to the recombinant virus of the invention.

### SUMMARY OF THE INVENTION

The present invention provides compositions comprising a novel recombinant virus which replicates selectively in cells or tissues that are hypoxic or have an activated HIF pathway. The novel compositions of the invention comprise a recombinant virus genetically engineered to have an hypoxia-responsive element, or a multiplicity of such elements, operably linked to a promoter which is operably linked to a gene or genes which regulate or modulate replication of the virus or encode a therapeutic molecule. Also included in the invention are constructs useful for screening for agents which interact with proteins or genes in the hypoxia-inducible pathway.

A first embodiment of the invention relates to compositions comprising a recombinant cytolytic virus which replicates selectivity in hypoxic cells or tissues or has an activated HIF pathway. The novel compositions of this embodiment comprise a recombinant virus genetically engineered to have an hypoxia-responsive element, or a multiplicity of such elements, operably linked to a promoter which is operably linked to a gene or genes which regulate or modulate replication of the virus, wherein the virus has a cytolytic cycle. The novel recombinant virus of this embodiment selectively replicates in and cytolyses hypoxic cells or tissues or has an activated HIF pathway. Examples of viruses that have cytolytic cycles include, but are not limited to, cytolytic adenoviruses, in particular adenovirus serotype 5; cytolytic picornaviruses, *e.g*., polioviruses; and cytolytic herpesviruses and herpes-like viruses, *e.g*., herpes simplex virus.

A second embodiment of the invention relates to compositions comprising an hypoxia HIF-dependent replicative virus that delivers a gene or genes selectively to cells that are hypoxic or have an activated HIF pathway. Compositions comprising the recombinant virus of this embodiment are genetically engineered to have an hypoxia-responsive element, or a multiplicity of such elements, operably linked to a promoter which is operably linked to at least one gene which regulates or modulates replication of the virus. The novel recombinant virus of this embodiment targets hypoxic tissues or cells, including tumors, where they selectively replicate and deliver a gene. According to this embodiment, an additional gene(s) is included in the novel recombinant virus of the invention which provides anti-angiogenesis activity or serves as a reporter gene or otherwise modulates hypoxic tissues or cells. An example of preferred genes that may be delivered by the novel compositions of the invention are angiogenesis inhibitory genes such as angiostatin.

A third embodiment of the invention relates to compositions comprising an hypoxia-dependent replicative oncolytic virus that delivers a gene or genes to cells that are hypoxic or have an activated HIF pathway. Compositions comprising the recombinant cytolytic virus of this embodiment are genetically engineered to have an hypoxia-responsive element, or a multiplicity of such elements, operably linked to a promoter which is operably linked to gene(s) which regulate or modulate replication of the virus, wherein the virus has a cytolytic cycle. The novel recombinant virus of this embodiment targets hypoxic tissues or cells, including tumors, where they selectively replicate, delivery a gene, and cause cytolysis. According to this embodiment, an additional gene(s) may be included in the novel recombinant cytolytic virus of this embodiment which provides anti-angiogenesis activity or serves as a reporter gene or otherwise modulates hypoxic tissues. An example of preferred genes that can be delivered by the compositions of this embodiment angiogenesis inhibitory genes such as angiostatin.

A fourth embodiment of the invention relates to a method of treating a condition or disease that is characterized by hypoxia. A preferred aspect of this embodiment relates to a method of treating an individual with cancer comprising administration of a recombinant replication-competent adenovirus that displays tumor cell specific lysis (oncolysis) and also delivers adjuvant therapy, in the form of an anti-angiogenic factor, to the tumor microenvironment. This is accomplished through the administration of a viral construct comprising an hypoxia/HIF-dependent replicative adenovirus (HYPR-Ad(s)) that expresses an anti-angiogenic factor under hypoxic conditions (HYPRA-Ad). In this method, the novel compositions of the invention have a synergistic effect in destroying hypoxic tumor tissues due to the effect of viral cytolysis and expression of an anti-angiogenic factor.

Another aspect of this embodiment of the invention relates to inducing activation of the hypoxia/HIF pathway in a tissue followed by treatment with the recombinant virus of the invention. In particular, undesired tissue, *i*.*e*., fat or scar, can be treated with an agent which specifically induces the HIF pathway in the undesired tissue. After treatement with the agent, the undesired tissue is susceptible to destruction by the recombinant virus of the invention.

In a fifth embodiment, the invention relates to compositions and methods useful for identifying compounds which modulate the hypoxia and HIF pathways. A stably or transiently transfected cell containing a vector comprising an hypoxia/HIF-inducible promoter operably linked to a reporter gene are contacted with a test compound and assayed for expression of the reporter gene. In a preferred embodiment, the HIF drug discovery assay is a high throughput assay suitable for large numbers of test compounds, *i*.*e*., by using libraries of compounds. Libraries of compounds are commercially available or may be synthesized using known procedures by a person of ordinary skill in the art. The drug discovery assays of the invention can also be used to test extracts of biological tissues, *i.e*., plant, fungal, bacterial, and animal.

In a sixth embodiment, the invention relates to drug discovery assays. A vector comprising an hypoxia- or HIF-inducible promoter operably linked to a reporter gene is used to generate a tumor containing the vector. The tumor containing the vector is used to assess the efficacy of a chemical or biological agent that specifically targets hypoxic tissue for destruction or disrupts the hypoxic induction pathway *in vivo.*

The invention also contemplates the use of genetic elements responsive to stimuli other than hypoxia, such as light (UV light, visible light), radiation (x-ray), pH, sound (radiowaves), redox status, metabolic status, hormonal response, and teleomere shortening.

### BRIEF DESCRIPTION OF THE FIGURES

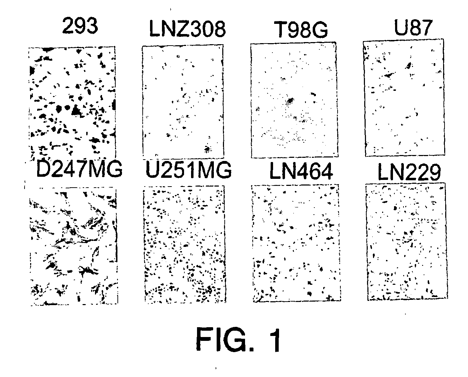

Figure 1: FIG. 1 shows the infection of tumor cells with recombinant adenovirus. The various cell lines indicated on the top of the panels were infected with recombinant adenovirus containing a LacZ reporter and stained for β-gal activity. See Example 1 for further description and experimental details.

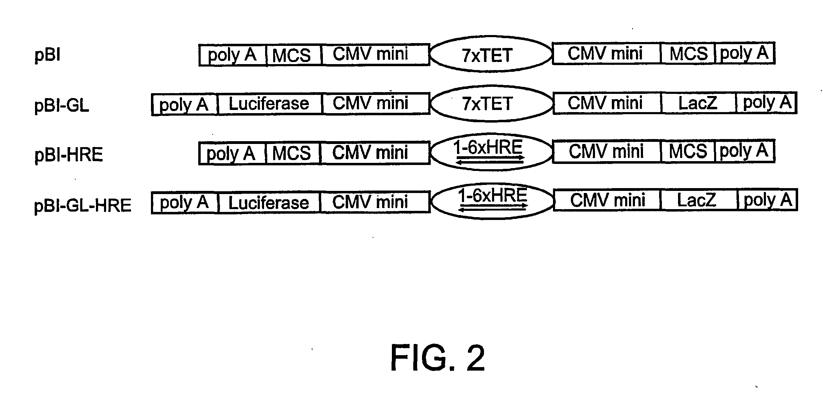

Figure 2: FIG. 2 shows schematic representations ofplasmids used to construct the novel recombinant viruses of the invention. MCS refers to multiple cloning site; TET-tetracycline responsive elements; HRE-hypoxia and/or HIF responsive elements; 1-6 refers to the number of tandem copies of HRE. See examples 4 and 5 for further description and experimental details.

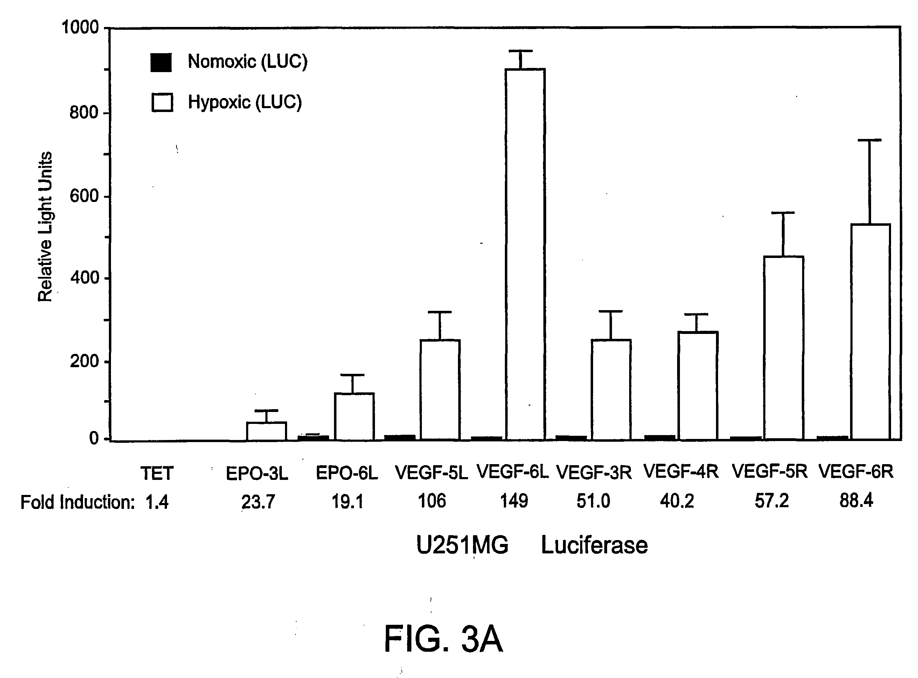

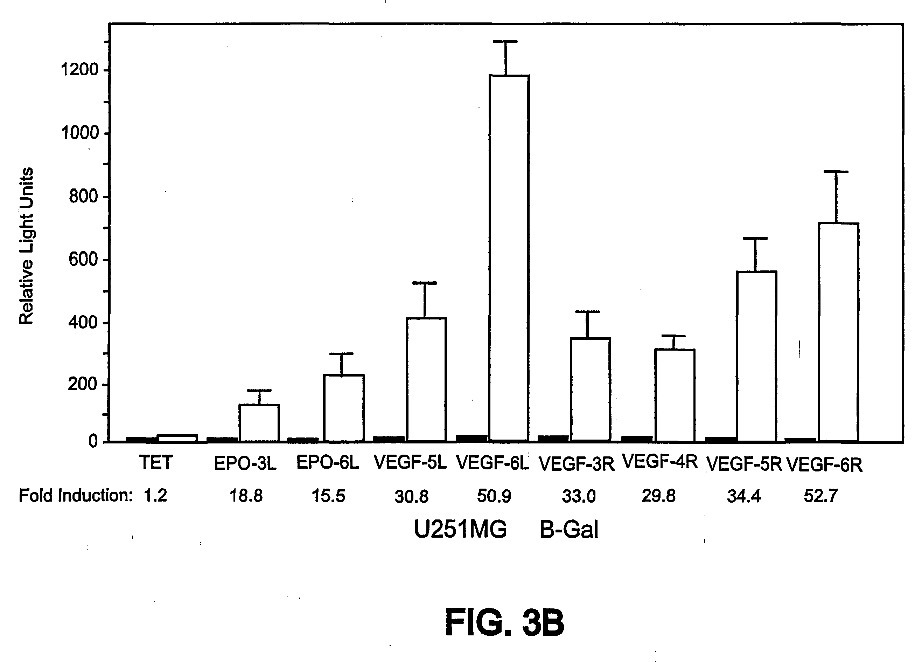

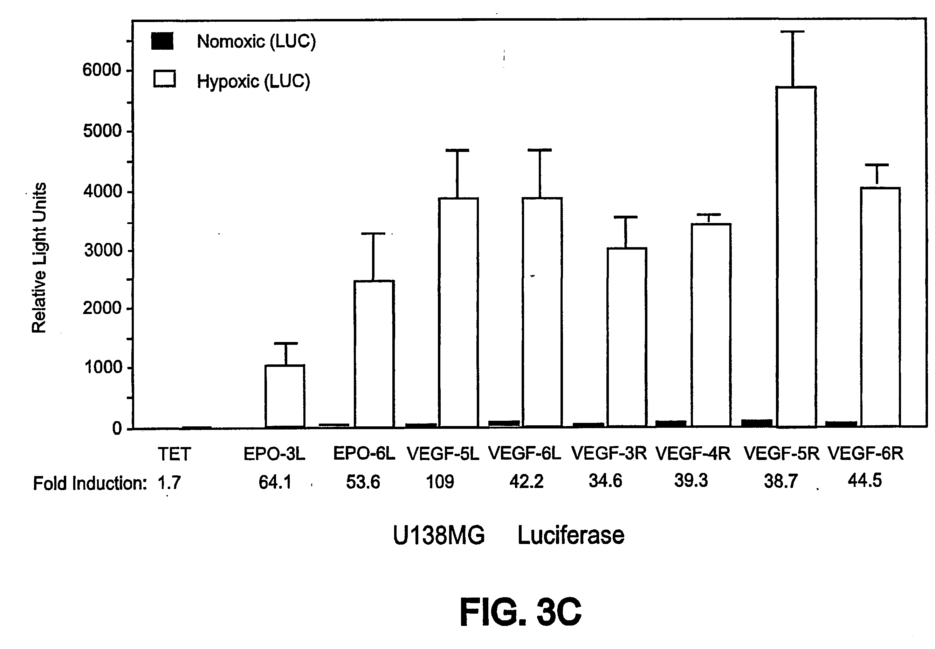

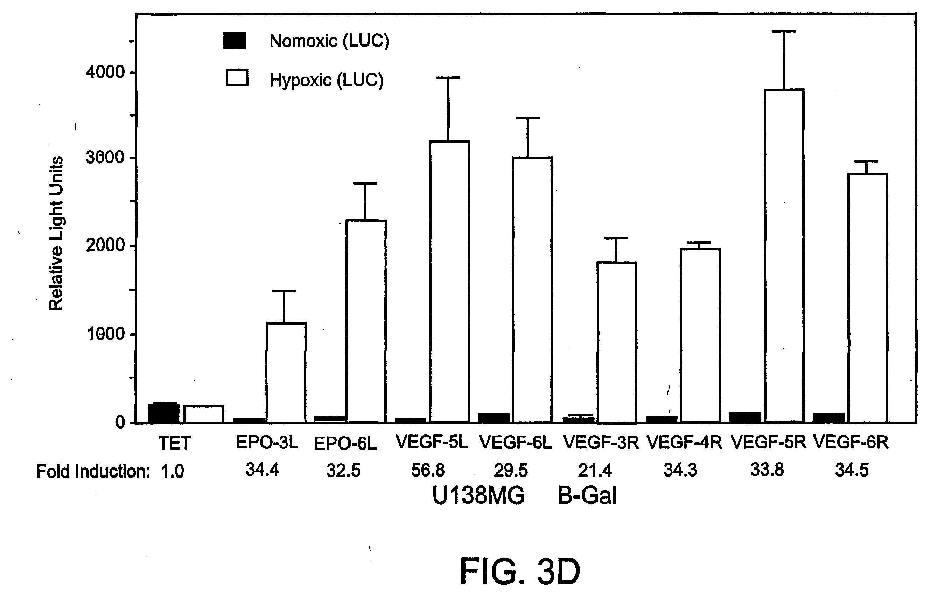

Figures 3A-D: FIGS. 3A-D shows the hypoxia induced expression of constructs in transfected tumor cell lines. VEGF or EPO-3,4,5,6 refers to the number of tandem repeats in the constructs, L for 5' orientation, and R for 3' orientation. FIGS. 3A and 3C shows ability of constructs to bi-directionally express luciferase in response to hypoxia; FIGS. 3B and 3D shows ability of construct to bi-directionally express β-Gal in response to hypoxia. See example 5 for further description and experimental details.

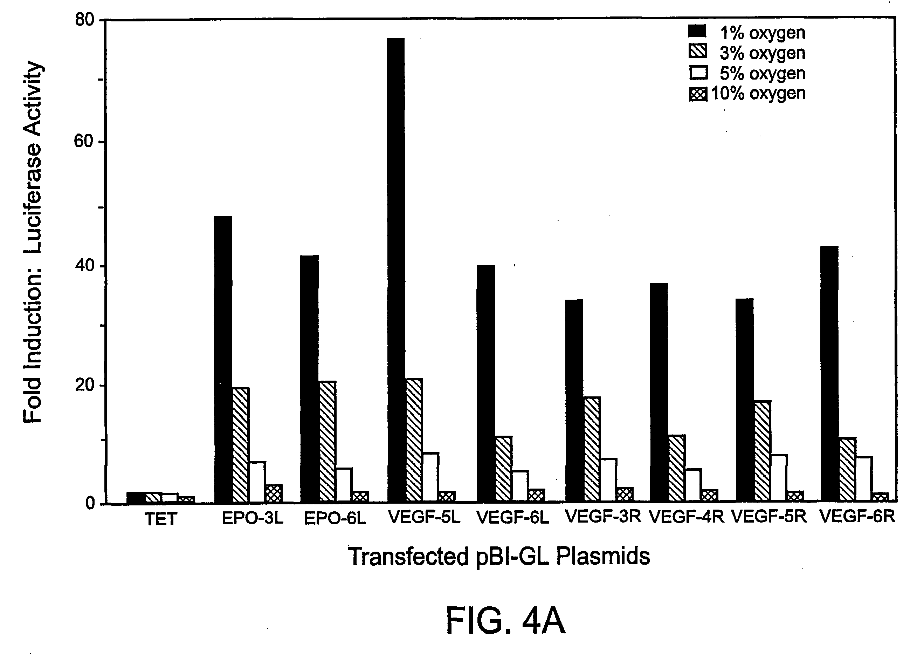

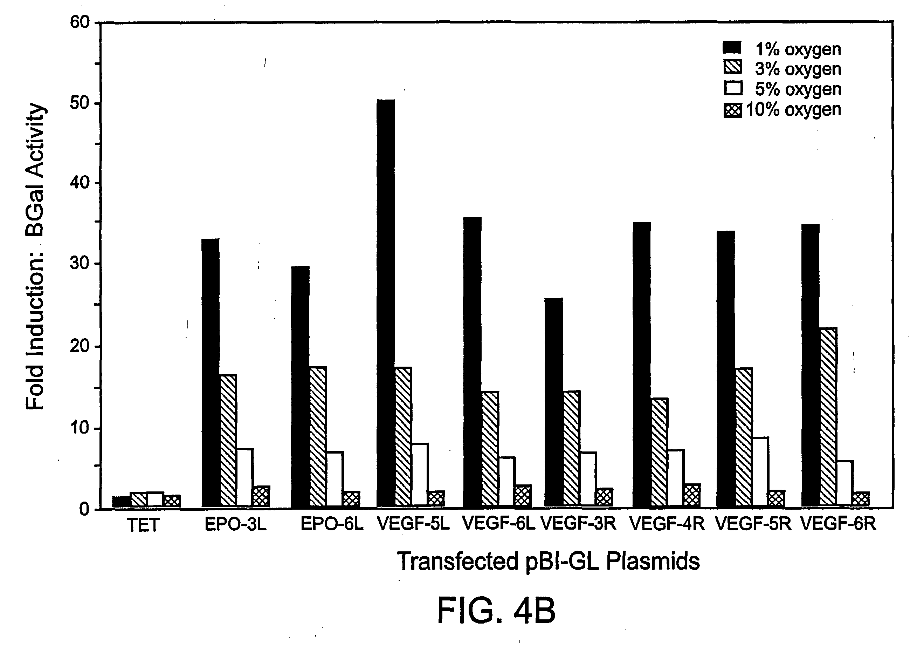

Figures 4A-B: FIGS. 4A-B shows the hypoxia induced expression of constructs in transfected tumor cell lines under variable oxygen partial pressure. VEGF or EPO-3,4,5,6 refers to the number of tandem repeats in the constructs, L for 5' orientation, and R for 3' orientation. FIG. 4A shows ability of constructs to bi-directionally express luciferase in response to variable oxygen partial pressures. FIG. 4B shows β-Gal bi-directional expression in response to variable oxygen partial pressures. See example 5 for further description and experimental details.

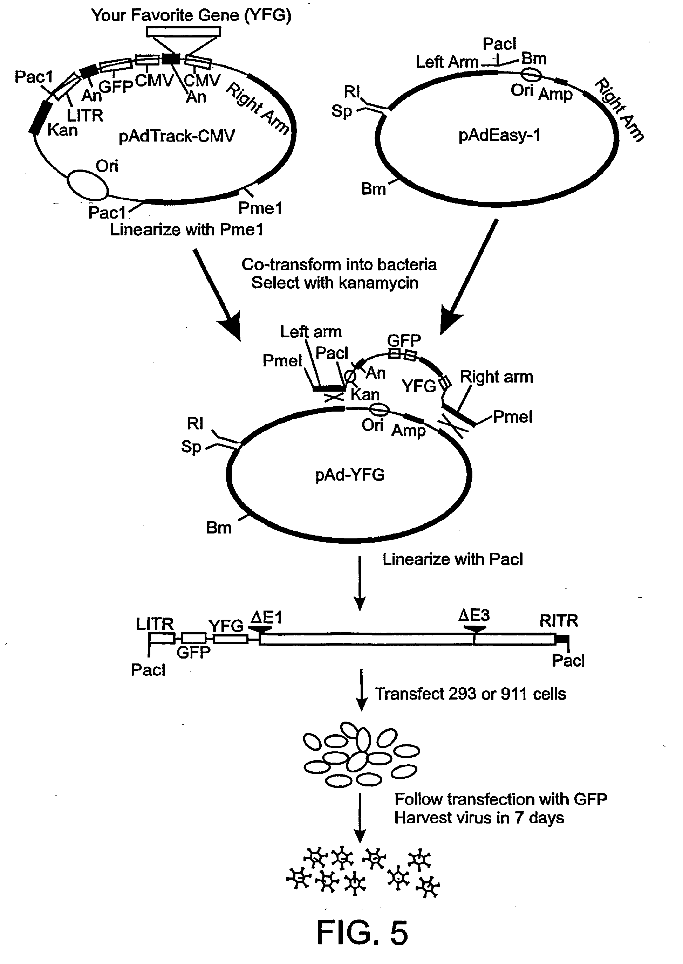

Figure 5: FIG. 5 shows a schematic outline for construction of recombinant viruses. See examples 6 -8 for further description and experimental details.

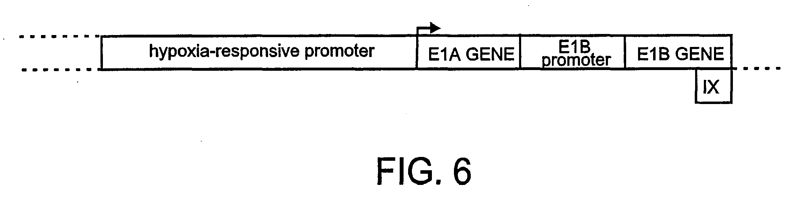

Figure 6: FIG. 6 shows a schematic representation of the subcloning of the E1 gene cassette into the pAdEasy adenoviral vector. See examples 6-8 for further description and experimental details.

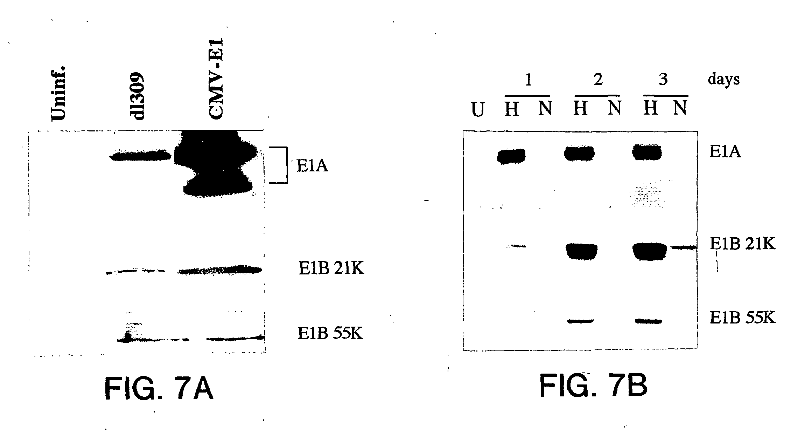

Figures 7A-B: FIGS. 7A-B shows expression of recombinant viral gene products in transfected tumor cell line (glioma LN229) by western blot analysis. In FIG. 7A, Uninf., is the uninfected cell line, dl309 is the cell line infected with adenovirus which is wild-type in the E1 region and has mutations in the E3 region, CMV-E1 is the construct containing the CMV minimal promoter and the E1 gene. In FIG. 7B U refers to uninfected cells, H refers to hypoxic conditions and N refers to normoxic conditions. See example 9 for further description and experimental details.

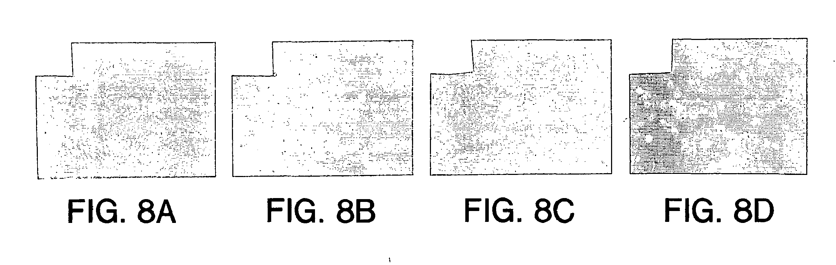

Figures 8A-D: FIGS. 8A-D shows the cytolysis of tumor cells in an hypoxia dependent manner. FIG. 8A is uninfected cells under hypoxia, FIG. 8B is Ad-CMV-E1 infected cells under normoxia conditions, FIG. 8C is HYPR-Ad1 under normoxia conditions, and FIG. 8D is HYPR-Ad1 under hypoxia. See example 10 for further description and experimental details.

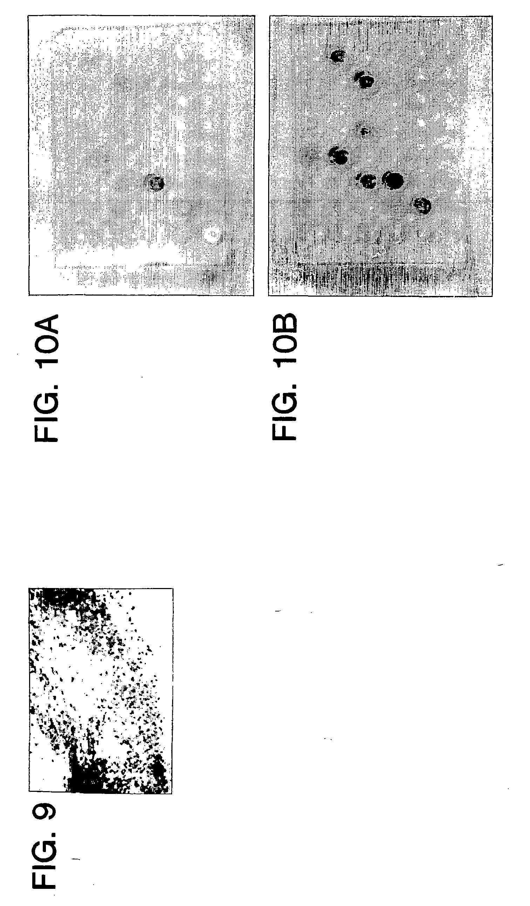

Figure 9: FIG. 9 shows the delivery of recombinant virus to brain tumors. Section of glioma from transplanted rat glioma tumor cells infected LacZ expressing replication-deficient adenovirus stained for β-gal expression. See example 11 for further description and experimental details.
Figure 10: FIGS. 10A-B show the results from assays for alkaline phosphatase enzyme activity on clones derived from a human glioma cell line stably transfected with a construct having an hypoxia responsive element operably linked to a promoter which is operably linked to the reporter gene alkaline phosphatase. FIG.10A shows cells exposed to normoxic conditions and FIG. 10B shows cells exposed to hypoxic conditions. See Example 12 for further description and experimental details.

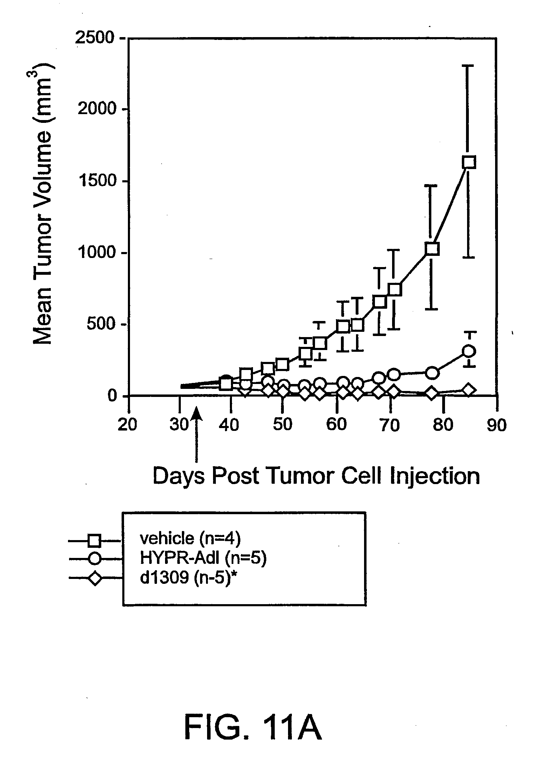

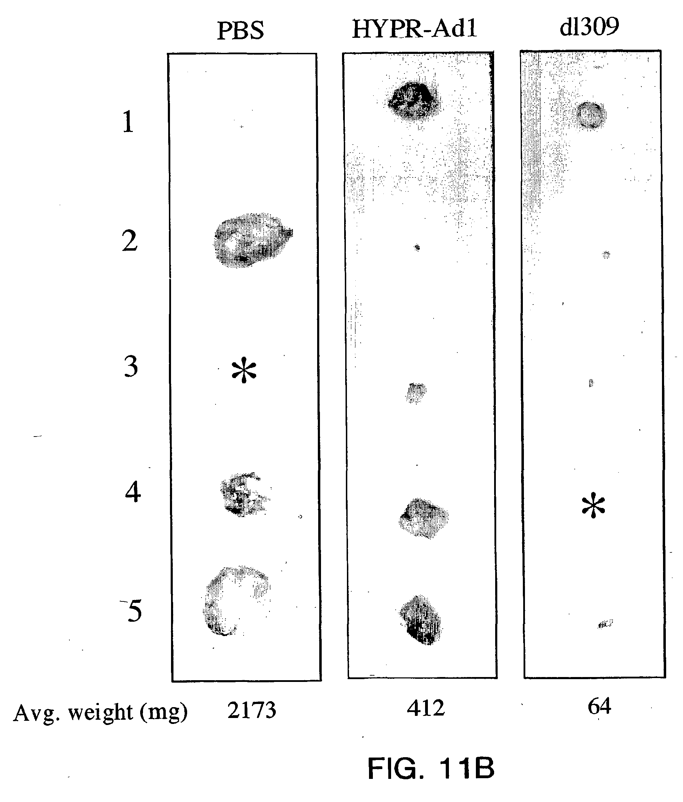

Figure 11: FIG. 11A is a graph showing the mean tumor volume of glioma cells in immunocompromised mice treated with HYPR-Ad1. FIG. 11B shows the size and weight of tumors with various treatments. The results shown suggest that HYPR-Ad1 reduces tumor growth by specifically causing cytolysis of infected hypoxic tumor cells. See Example 15 for further description and experimental details.

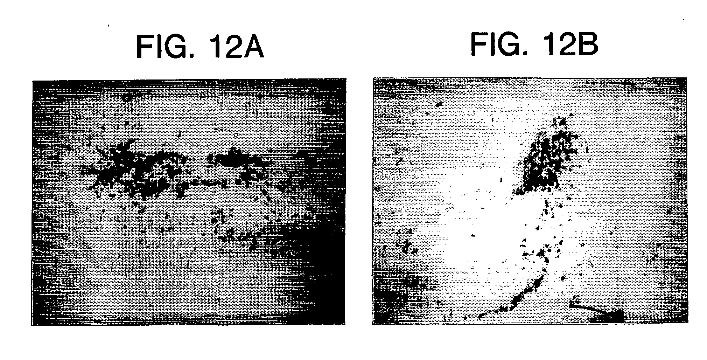

Figure 12: FIGS. 12A and 12B show *in vivo* evaluation of compounds modulating expression of hypoxia. See Example 14 for further description and experimental details.

### DETAILED DESCRIPTION OF THE INVENTION

The present invention was based on the discovery of the inventors that viruses genetically engineered to have a protein essential for viral replication under the control of an hypoxia and/or HIF responsive element/promoter construct selectively target and cytolyse hypoxic tissues. The inventors further discovered that the novel recombinant viruses can be further engineered to selectively deliver a gene for diagnostic or therapeutic purposes to hypoxic tissues or cells.

The present invention provides compositions comprising recombinant viruses which replicate selectively in hypoxic cells. The novel compositions of the invention comprise recombinant viruses genetically engineered to have an hypoxia-responsive element operably linked to a promoter which is operably linked to a gene or genes which regulate or modulate replication of the virus. The compositions comprise recombinant viruses that may or may not have a cytolytic cycle. Viruses with a cytolytic cycle are preferred. The novel recombinant viruses of the invention selectively target any hypoxic tissues or cells that are hypoxic or in which the HIF pathway has been activated. Tumors that are hypoxic or have regions of hypoxia are preferred targets for the compositions of the invention.

A preferred embodiment of the invention relates to a recombinant replication-competent adenovirus that displays tumor cell specific lysis (oncolysis) and also delivers adjuvant therapy, in the form of an anti-angiogenic factor, to the tumor microenvironment. This is accomplished through the administration of a viral construct comprising an hypoxia-dependent replicative adenovirus (HYPR-Ad(s)) that expresses an anti-angiogenic factor under hypoxic conditions (HYPRA-Ad).

Another preferred embodiment of the invention relates to a method of treating a cancer patient with a tumor that is hypoxic, has an activated HIF pathway, or has regions of hypoxia/HIF activity by administering the composition of the invention. The method ofthe invention allows for the treatment ofpatients, including humans and animals, with a wide variety of tumors, so long as the tumors are hypoxic or have regions of hypoxia. A specific method of this embodiment is directed to cancer patients that have tumors which were previously treated with chemo- or radiotherapeutic techniques and have become resistant to such treatments. Such chemo-and radiotherapeutic techniques are known to kill or destroy non-hypoxic tumor tissues, thereby allowing for survival of hypoxic tumor tissue. Thus the methods of the invention are suited for treating patients that have received previous or are currently receiving chemo- and radiotherapeutic treatments.

As used herein the term "normoxia" or "normoxic" refers to a normal level of oxygen or oxygen tension in a cell or tissues.

As used herein the term "hypoxia" or "hypoxic" refers to a lower level of oxygen or oxygen tension in a cell or tissue compared to what is normally found. Cells or tissues are hypoxic when the O₂ concentration is lower than the normal level of oxygen in these particular cells or tissues.

As used herein the term "tumor hypoxia" or "hypoxic tumor cells" refers to a physiological difference in oxygen levels between normal and tumor tissue wherein the partial pressure of oxygen is reduced in the tumor tissue as compared to the normal tissue.

As used herein the term "cells or tissues with activated HIF" refers to cells or tissues in which the HIF transcription factor pathway is either constitutively active or in which it was activated by an exogenous stimulus or treatment. A number of agents are known to those of ordinary skill in the art to activate the HIF pathway and include, but are not limited to, iron chelators, cobalt, proteosome inhibitors, and galdanamycin.

As used herein the term "oncolytic" or "oncolysis" refers to the ability lyse or destroy cancer or tumor cells.

As used herein the term "cytolytic" or "cytolysis" or "cytolyse" refers to the ability to lyse or destroy a cell or cells.

As used herein the term "anti-angiogenic" or "anti-angiogenesis" refers to the capability of inhibiting angiogenesis. Inhibitors of angiogenesis may be a gene or a protein which acts either directly or indirectly to modulate angiogenesis. Inhibitors of angiogenesis include, but are not limited to, angiostatin, anti-angiogenic peptides, anti-angiogenic antisense DNA, and other anti-angiogenic factors known to those skilled in the art.

As used herein the term "anti-tumor" or "anticancer" refers to the capability to destroy or reduce the size of a tumor or cancerous growth.

### Drug Discovery Assays

In one embodiment, the invention relates to a method of identifying a compound which modulates the hypoxia-inducible pathway mediated induction of protein and/or gene expression. In this method, a cell line stably or transiently transfected with a vector comprising an hypoxia-inducible promoter operably linked to a reporter gene is used to detect compounds which modulate the expression of genes or any proteins modulated by hypoxia and/or HIF. In one aspect of this method, cell lines comprising an hypoxia-inducible promoter operably linked to a reporter gene can be used to detect compounds which inhibit expression of the reporter gene. It is preferred that expression of the reporter gene can be readily detected, *e.g*., by a simple colorimetric assay. Other genes which can be detected by other techniques such as enzymatic or fluorometric assays can be used as the reporter gene.

Compounds that test positive in the drug discovery assays of the invention are those that modulate the expression of the reporter gene. For example, cells are incubated with a test compound under specified conditions and compared to cells incubated under identical conditions except for the absence of that compound. A comparison between reporter gene expression with the test compound and reporter gene expression from the no-compound assay allows one to determine if the test compound is positive. Those test compounds which alter expression levels of the reporter gene compared to the no-compound (or other appropriate control) have tested "positive."

Materials that test positive in the drug discovery assays of the invention are useful for modulating the hypoxia/HIF pathway which is associated with a variety of clinical significant conditions, *i.e*., cancer, ischemia, and the like.

In certain aspects of this embodiment, the cell line is lysed or further processed before it is contacted with the test compound. In any case, after the test compound is incubated for a selected period of time with the cell or portions thereof, the reaction mixture is assayed for level of reporter gene expression. In particularly useful aspects of this embodiment, the reporter gene expresses a protein that is readably detectable, *e.g*., an enzyme which catalyzes a reaction that is detected by a simple colormetric assay or by other means such as monoclonal antibody detection. Examples of reporter genes useful in the invention include, but are not limited to, luciferase, β-galactosidase alkaline phosphatase, green fluorescent protein, etc.

In a specific example, a vector comprising an hypoxia and/or HIF inducible promoter operably linked to a gene for alkaline phosphatase is stably or transiently transfected into a cell line. The cell lines comprising the vector are subjected to conditions which activate the hypoxia-inducible factor pathway and are contacted with a test compound. In general, when a test compound reduces or increases expression of alkaline phosphatase activity, it is identified as an hypoxia- and/or HIF-inducible pathway modulator. Test compounds which modulate expression of alkaline phosphatase activity have tested "positive" and are examined further.

The invention also relates to compounds and compositions comprising the compounds that test positive in the drug discovery assays of the invention.

### Viruses

In general any virus may used in the invention. Selection of the appropriate virus depends on a variety of factors such as the host to be treated. For example, treatment of a human requires a virus which can infect and replicate in *Homo sapiens*. Viruses that can be used to treat non-human subjects such as other mammals are also encompassed within the scope of the invention. Preferred viruses have a cytolytic cycle, *i.e*., the ability to lyse cells. Preferred cytolytic viruses are those of the adenovirus family. Examples of viruses from the adenovirus family include, but are not limited to, Adenovirus types 1-41, as described in the Catalogue of Animal Viruses & Antisera, Chlamydias & Rickettsias 6th edition, 1990 from the American Type Culture Collection (ATCC). Other viruses suitable for use in the invention include, but are not limited to papillomaviruses, retroviruses, picornaviruses, *e.g*., polioviruses; herpesviruses and herpes-like viruses, *e.g.,* herpes simplex virus; and others described in Catalogue of Animal Viruses & Antisera, Chlamydias & Rickettsias 6th edition, 1990 from the American Type Culture Collection.

### Recombinant Viruses

In general the recombinant viruses of the invention are genetically engineered to replicate selectively under hypoxic conditions. Recombinant viruses ofthe invention can be constructed by identifying genes that are responsive to oxygen partial pressures, i. e., genes that contain hypoxia responsive elements or hypoxia-inducible enhancer motifs (HRE). Using standard genetic engineering methods, any suitable promoter can be linked to HRE, which are then linked to a gene(s) in a particular virus that regulates or modulates virus replication. A variety of genes and/or their products are known to those skilled in the art that regulate or modulate viral replication. For example, the E1A gene product is known to encode an early viral protein essential for initiation of adenovirus replication. Thus this E1A gene of an adenovirus (or any structural or functional homolog) may be engineered to be put under the control of an hypoxia responsive element/promoter, thus creating an organism that selectively replicates under hypoxic conditions.

Hypoxic conditions are known to initiate a cascade of physiological responses and lead to the induction of genes involved in glycolysis, erythropoiesis, and angiogenesis. The HIF-1 protein complex, which is a heterodimer composed of the two basic helix-loop-helix proteins HIF-1α and HIF-1β, mediates transcriptional responses to hypoxia by binding to cis-acting hypoxia-inducible enhancer motifs (HRE) present within target genes. In addition to HIF-1, other members of the hypoxia-1 inducible pathway include HIF-2 and HIF-3. The HRE present within the 3'-flanking region of the erythropoietin (EPO) gene and 5'-flanking region of the VEGF gene are less than 50 bp in length. These HRE's contain highly conserved HIF-1 binding sites and other gene-unique cis-acting sequences that are functionally essential for hypoxic induction. EPO is a glycoprotein hormone produced in the kidney and liver in response to hypoxia and stimulates erythropoiesis by binding to its receptor expressed on erythroid progenitor and precursor cells. EPO and its receptor are also expressed in the central nervous system by astrocytes and neurons, respectively, where it is currently believed that they act in a paracrine fashion and function to protect neurons against hypoxia-induced damage. VEGF is induced by hypoxia in a variety of cell types and is also expressed by a large number of tumor cell types, including gliomas. VEGF is a major regulator of angiogenesis and has mitogenic activity that is specific for vascular endothelial cells. Based on this information, EPO and VEGF HRE's were chosen for the design and testing of a hypoxia-responsive promoter.

### Gene control replication

Adenoviruses are DNA viruses that infect both dividing and quiescent cells. Re-entry of infected quiescent cells into the cell cycle is required for viral DNA replication and ultimately, viral progeny production. The expression of E1A gene products is essential for these viral functions and adenoviruses which lack the E1A gene region are replication-deficient. The adenoviral E1A gene is the first transcription unit to be expressed from a constitutively active promoter region. Products of the E1A gene exhibit a wide range of biological activities including the modulation of cellular and viral transcription (including the induction of E1B gene transcription) and the induction of DNA synthesis in quiescent cells. However, deregulation of cell growth control by E1A induces apoptosis through p53 dependent and independent mechanisms and ultimately interferes with viral progeny production. The prevention of apoptosis during wild type adenovirus infection is mediated by expression of the adenoviral E 1 B gene products. The E1B gene encodes two proteins, 21 K and 55K, which function independently to inhibit E1A-induced apoptosis. The E1B 21K protein is homologous in sequence and function to the Bcl-2 family of apoptosis regulators and blocks E1A induced apoptosis as well as many other apoptotic stimuli. The infection of cells with adenoviruses lacking E1B 2 1 K function leads to the appearance of extensive nuclear and viral DNA degradation (deg phenotype) and enhanced cytopathic effect (cyt phenotype). The E1B 55K, in conjunction with the adenoviral E4-orf6 gene product, has two functions during viral production: to directly interact with and inactivate p53, and later in viral production to facilitate the transport of viral late mRNA while inhibiting the transport of most cellular mRNA.

Recombinant viruses of the invention can be further engineered to contain a gene that allows for the termination of viral propagation with an exogenous agent, such as thymidine kinase, which would render them susceptible to ganciclovir. In addition, recombinant viruses can be further engineered so that the number of genes expressed is increased to four if internal ribosomal entry sites (1RES) are used to express two genes per transcription unit. According to this embodiment, a plurality of genes can be expressed in response to hypoxia or under conditions which activate the HIF-1 pathway.

### Tumors and Hypoxia

Tumors, including, solid tumors are physiologically distinct from surrounding normal tissues and the tissues from which they are derived (Brown and Giaccia, 1998 Cancer Res. 58:1408-1426). An underlying difference in tissue vasculature between normal tissue and cancer tumors leads to the unique physiological characteristic of poor oxygenation, or hypoxia, in tumors. Tumor blood vessels are highly abnormal as a result of (1) the invading process of tumor cells on tissues containing normal vasculature and (2) the release of angiogenic factors by the tumors. Blood flow in solid tumors is often sluggish and leakier than that seen in normal tissues and is due to the abnormal, anfractuous nature of the tumor blood vessels. It is believed that these abnormal characteristics of tumor vascularization lead to the hypoxic physiological state of tumor tissues. A large body of evidence has suggested that hypoxic tumors are more resistant to radio- and chemotherapeutic treatments (See, *e.g*., Gray et al. 1953 Br. J. Radiol. 26:638-648; Teicher et al. 1990 Cancer Res. 50:3339-3344; Grau and Overgaard 1988 Radiother. Oncol. 13 : 3 01-3 09).

Regions of hypoxia in tumors have been shown to occur in many solid tumor model systems, see, *e.g*., Gullino, P. M., et al., Adv. Exp. Med. Biol. 75:521-536 (1975); Hasegawa, T., et al., Int. J. Radiat. Oncol. Biol. Phys. 13:569-574 (1987); Jain, R., et al., Cancer Res. 48:2641-2658 (1988); Siemann, D., et al., Br. J. Cancer 58:296-300 (1988); Song, C., et al., Cancer Res. 47:442-446 (1987); Vaupel, P., et al., Cancer Res. 47:3496-3503 (1987); Vaupel., P., et al., Cancer Res. 41:2008-2013 (1981). Tumors can also have an activated HIF pathway independently of hypoxias. Tumors associated with the Van Hippel Lindau syndrome comprising haemoglioblastoma, clear cell renal and carcinoma pancreatic and inner ear tumors are examples.

A variety of methods are available and known to one of skill in the art for detecting hypoxic tissues. See, for example, Chapman, J.D., "The Detection and Measurement of Hypoxic Cells in Solid Tumors", Cancer, vol. 54, No. 11, pp. 2441-2449 (1984). Chapman, J.D., "Measurement of Tumor Hypoxia by Invasive and Non-Invasive Procedures: A Review of Recent Clinical Studies", Radiother. Oncol., 20, pp. 13-19 (1991); "Development of F-18-labeled fluoroerythronitroimidazole as a PET agent for imaging tumor hypoxia" Yang DJ, Wallace S, Cherif A, Li C, Gretzer MB, Kim EE, Podoloff DA. Radiology 194:795-800,1995. U.S. PatentNos. 5,401,490 and 5,843, 404 disclose methods of detecting hypoxia or hypoxic tissues. Any of these techniques or others known to those skilled in the art may be used to identify hypoxic tissues.

### Angiogenesis

An essential component of tumor growth is angiogenesis, the establishment of new blood supply from preexisting vessels. Tumors need to disrupt physiological controls over angiostasis to initiate neovascularization, a process triggered by the release of hypoxia-inducible angiogenic factors by tumors when they reach about 0.4 mm in diameter. Angiogenesis is a stepwise process during the malignant progression of astrocytoma and other cancers. In the development of gliomas, new blood vessels appear in low grade astrocytoma followed by an increase in density in anaplastic astrocytoma. During the transition from anaplastic astrocytoma to glioblastoma, extensive microvascular proliferation occurs, leading to abnormal vessels. Hypoxia is an integral component of astrocytoma progression and necrosis develops in its ultimate phase. Vascularity and microvascular cell proliferation are morphological features used to diagnose malignant astrocytomas from their less malignant counterparts and these features correlate with prognosis.

Targeting the vascular component of human tumors, including gliomas, provides a particularly effective cancer therapy because: i) it is estimated that about 100 tumor cells would be affected by the killing of each endothelial cell; ii) it is less likely that endothelial cells would become resistant to the treatment since they do not share the genetic instability of tumor cells; and, iii) strategies interfering with tumor vasculature have been used with success in animal models

A variety of anti-angiogenesis agents can be used to enhance the effect of the recombinant virus of the invention. One of these inhibitors, angiostatin (see, *e.g.,* U.S. patent no. 6,024,688, and O'Reilly et al., "Angiostatin: A Circulating Endothelial Cell Inhibitor That Suppresses Angiogenesis and Tumor Growth", Cold Spring Harbor Symposia on Quantitative Biology, vol. LIX, pp. 471-482 (1994)), was used effectively against a variety of murine and human xenotransplanted tumors including gliomas, breast, prostate, and lung carcinomas. Moreover, angiostatin potentiates the anti-tumor effects of ionizing radiation by a combined cytotoxic effect on endothelial cells. The potent antitumor properties of angiostatin for use as a local adjuvant therapy can be delivered by the hypoxia-dependent oncolytic adenovirus of the invention.

Other angiogenesis inhibiting molecules include members ofthe thrombospondin (TSP) family of proteins. (See, *e.g.,* de Fraipont et al., 2001, Trends. Mol. Med. 7(9):401-467.) Several members of this family, TSP1 and TSP2, are known to have anti-angiogenesis activity. The anti-angiogenesis activity of the TSPs is localized to their procollagen and type 1 repeat (TSR) domains, a feature which other members of the family do not have. Thus, the recombinant virus of the invention can be engineered to have a TSP gene or a portion thereof under control of the hypoxia responsive element. Since the anti-angiogenic activity is localized to a small portion of the protein, nucleic acids encoding the anti-angiogenic activity can be used in the invention.

Other angiogenesis inhibiting factors containing TSR domains that may be used in the invention are GD-A1F (see, *e.g*., PCT/US95/02634 ). BAI1, BAI2 and BAI3, and members of the ADAMTS family of proteins including, but not limited to, ADAMTS-1, 4, and 8 (see, *e.g*., U.S. patent 6,046,031). The number of anti-angiogenic genes is growing and any of these could be used in the invention.

Other genes which can be used in the invention include, but are not limited to, endostatin (see, *e.g*., U.S. patents 6,174,861 and 5,854,205), platelet factor-4 (PF4) (see, *e.g.,* U.S. patent 5,482,923), interleukin-4 (IL-4) (see, *e.g.,* U.S. patents 5,382,427 and 4,958,007), and pigment epithelium-derived factor (PEDF) (see, *e.g*., U.S. patents 6,288,024 and 5,840,686). Genes encoding brain angiogenesis inhibitors (1,2,3), interleukin-12, tissue inhibitors of metalloproteinases, prolactin (10 kD fragment), bFGF soluble receptor, transforming growth factor beta, interferon alpha, placenta proliferin related protein, dominant negative fragments of vascular endothelial growth factor receptor or fragments thereof can also be used in the invention.

### Antitumor Agents

A variety of genes which exhibit antitumor activity or enhance the antitumor activity of the virus or modulate an immune response against a tumor may be incorporated into the novel viral constructs of the invention. These antitumor agents operate by a variety of mechanism. For example, Bacillus thuringiensis subspecies thuringiensis has a protein named oncotoxin, which has antitumor activity (U.S. patent no. 5,977,058). The genes for proteins such as this may be genetically engineered into the novel recombinant viruses of the invention to enhance their antitumor activity. Other examples of antitumor agents include, but are not limited to, the various tumor suppressor genes that are know to those skilled in the art.

### Other Agents

Other agents can be delivered by the recombinant virus of the invention for therapeutic purposes and include, but are not limited to, modulators of cell proliferation, cell cycle, cell growth, cell motility, apoptosis, immune response, metastasis, as are known to those of ordinary skill in the art.

### Other disorders

Hypoxic or HIF deregulation is associated with a number of mammalian diseases including, but not limited to arthritis, diabetic retinopathy, ischemic heart disease, stroke, tumors and pregnancy disorders (preclampsia and intrauterine growth retardation). The invention can be utilized to deliver a therapeutic gene(s) in such conditions. Moreover, it can be used to generate transgenic animals containing the reporter system for testing a variety of therapeutic agents to treat the aforementioned conditions.

### Routes of Administration and Dosages

The novel compositions of the invention can be administered through a variety of routes including, but not limited to, subcutaneously, intraperitoneally, intravenously, etopically, through aerosols, and intracerebraly. Routes of administration are known to those skilled in the art.

The novel recombinant viruses of the invention can be administered in a single dose or in multiple doses and more than one tumor in an individual needing treatment can be treated concurrently.

It should be understood that the detailed description and specific examples, while indicating preferred embodiments of the invention, are given by way of illustration only, since various changes and modifications within the spirit and scope of the invention will become apparent to those skilled in the art from the detailed description and specific examples.

### EXAMPLES

### Example 1: Infection of tumor cell lines by adenoviruses

This example shows brain tumor cell lines are efficiently infected by adenoviruses. The utilization of adenoviruses for gene therapy treatment of brain tumors is dependent upon viral entry. Infection of human cells with adenoviruses is a multistep process which involves the specific interaction of at least two viral proteins and their respective cellular receptors. Brain tumors are histologically and genetically heterogeneous and, therefore, it is possible that only a small subset of these tumors can be infected by adenoviruses. To test this, the capacity of 8 human glioma cell lines (D247MG, U251MG, LN-229, LN-464, U87MG, LN-Z308, T98G, U138MG) to be infected by adenovirus was examined using a replication-deficient adenovirus which contains a constitutively active exogenous LacZ reporter gene (AdLacZ, UNC-Virus Vector Core Facility, Chapel Hill, NC). Uninfected glioma cells and AdLacZ infected 293 human embryonic kidney cells were used as negative and positive controls, respectively.

Equal numbers of each cell line were seeded 24 h prior to infection. The cells were infected with AdLacZ at multiplicities of infection (MOI) ranging from 0.1-500 or were mock infected. 24 h post infection, cells were histochemically stained for β-galactosidase (β-gal) activity using the X-gal substrate and the number of infected (blue) cells at each MOI was visually quantified The results are summarized in FIG.1. Five of the eight cell lines achieved 75-100% infectivity at a MOI ranging from 100 to 500 while the remaining three cells lines (LN-Z308, T98G, U138MG) infected less efficiently (<30%) even at an MOI of 500. The 293 cells infected at very high efficiency with the AdLacZ virus attaining 50% infection at a MOI of 1 and greater than 95% infection at a MOI of 5.

These results establish that a large subset (more than about 60%) of brain tumor cell lines can be infected by an adenovirus at high frequency. The differential susceptibility of human cancer cell lines to adenovirus infection is similar to that in other cell types, including human bladder and colon cancer cell lines.

### Example 2: Effect of hypoxia on adenovirus infection of tumor cell lines

This example shows that adenovirus infection of brain tumor cell lines is not affected by hypoxia. The therapeutic efficacy of the HYPR adenovirus is dependent upon its ability to efficiently infect hypoxic tumor cells. The level of adenovirus infection under normoxic and hypoxic conditions in two genetically and biologically diverse glioma cell lines, D247MG and LN-229, were compared. The cells were incubated for 72 h under normoxic (20.8% O₂) or hypoxic (1% O₂) conditions and then infected with a replication-deficient AdLacZ virus at several MOI's as described above. The cells were histochemically stained at 24 h post infection for β-gal activity and the percentage of infected (blue) cells was visually quantified (Table 1). It was found that the percentage of infected cells was slightly reduced under hypoxic conditions in D247MG (9% reduction) and LN229 (28% reduction) cells at subsaturating MOI's. This was overcome by increasing the MOI to 250 for LN-229, yielding 100% infected cells under normoxia and hypoxia. These experiments demonstrate that adenovirus infection under hypoxic conditions in cell culture occurs at levels similar to that under normoxic conditions. Thus, this efficiency permits virus multiplication and propagation since it is estimated that each infected cell will produce up to 10³-10⁴ new viral particles.

**Table 1: Percent blue cells/field**

| | D247MG Cells | LN-229 Cells |
|---|---|---|
| Normoxia | 35 (+/- 6) | 78 (+/- 6) |
| Hypoxia | 32 (+/- 4) | 56 (+/- 8) |

### Example 3: Replication and progeny production of adenovirus under hypoxic conditions

This example shows AdLacZ replication and progeny production is efficient under hypoxic conditions. The possibility that hypoxic cells will not allow high efficiency adenovirus replication and progeny production was tested in 293 cells using AdLacZ. The 293 cells are a human embryonic kidney cell line which complements, in trans, the viral functions missing in replication-deficient AdLacZ and allows viral production. Viruses were harvested from AdLacZ infected 293 cells grown under normoxic (20.8% O₂) and hypoxic (1% O₂) conditions and then used to infect D247MG and LN-229 glioma cell lines under normoxia at several different MOI's. The cells were histochemically stained at 24 h post infection for β-gal activity and the percentage of infected (blue) cells was visually quantified and used as an estimate of viral titer. The results are summarized in Table 2. It was found that the percentage of infected (blue) cells was reduced by approximately 20% when virus was produced in hypoxic 293 cells. These results suggest that viral production is only slightly reduced in hypoxic 293 cells.

**Table 2**

| | D247MG Cells | | LN-229 Cells | |
|---|---|---|---|---|
| | % Blue cells/field | Inferred viral titer (10⁸ pfu/ml) | % Blue cells/field | Inferred viral titer (10⁸ pfu/ml) |
| Normoxia | 61 (+/- 8) | 1.1 | 62 (+/- 3) | 2.9 |
| Hypoxia | 49 (+/- 5) | 0.9 | 56 (+/- 8) | 2.6 |

In summary, the experiments described in Examples 1-3 demonstrated that a large subset of glioma cell lines can be infected with an adenovirus at high efficiency and that adenoviral infection, replication, and progeny production are not dramatically altered by hypoxic conditions.

### Example 4: Construction of an hypoxia responsive promoter

Example 4 describes the design and testing of an hypoxia-responsive promoter to be used for the generation of an hypoxia- and/or HIF-dependent replicative adenovirus (HYPR-Ad's). The construction of the HYPR (hypoxia and/or HIF regulated) series of recombinant adenoviruses requires the hypoxia/HIF-dependent regulation of the

| | | | | |
|---|---|---|---|---|
| ANTI-ANGIOGENIC GENE | MINIMAL PROMOTER | HYPOXIA-RESPONSE ELEMENTS | MINIMAL PROMOTER | E1 GENE |

A commercially available mammalian expression vector (pBI, Clontech, Palo Alto, CA, FIG. 2) was used as a the foundation for the generation and testing of a bi-directional hypoxia/HIF-responsive promoter. The pBI plasmid contains a tetracycline-responsive element which allows conditional bidirectional expression of two heterologous genes. Hypoxia-responsive elements were identified in several genes and appeared to function as classical enhancer elements. Therefore, these elements were selected for these constructs to function bidirectionally to co-regulate the expression of two genes.

The first step in the construction of the HYPR virus was the selection of the hypoxia/HIF-responsive promoter. Hypoxic conditions are known to initiate a cascade of physiological responses and lead to the induction of genes involved in glycolysis, erythropoiesis, and angiogenesis. The HIF-1 protein complex, which is a heterodimer composed of the two basic helix-loop-helix proteins HIF-1α and HIF-1β, mediates transcriptional responses to hypoxia by binding to cis-acting hypoxia/HIF-inducible enhancer motifs (HRE) present within target genes. As part of the family of hypoxia-activatcd transcription factors, HIF2 and HIF3 have also been identified and found to be functionally similar to HIF1α. The HRE present within the 3'-flanking region of the erythropoietin (EPO) gene and 5'-flanking region of the VEGF gene are less than 50 bp in length. These HRE's contain highly conserved HIF-1 binding sites and other gene-unique cis-acting sequences that are functionally essential for hypoxie/HIF induction. EPO is a glycoprotein hormone produced in the kidney and liver in response to hypoxia and stimulates erythropoiesis by blinding to its receptor expressed on erythroid progenitor and precursor cells. EPO and its receptor are also expressed in the central nervous system by astrocytes and neurons, respectively, where it is currently believed that they act in a paracrine fashion and function to protect neurons against hypoxia-induced damage. VEGF is induced by hypoxia in a variety of cell types and is also expressed by a large number of tumor cell types, including gliomas. VEGF is a major regulator of angiogenesis and has mitogenic activity that is specific for vascular endothelial cells. Based on this information, EPO and VEGF HRE's, whose sequences are known (Semenza et al. 1995, Chest 114:40S-45S) were chosen for the design and testing of an hypoxia/HIF-responsive promoter.

The EPO HRE sequence, SEQ ID NO:1, GCCCTACGTG CTGTCTCACA CAGCCTGTCT GAC, and the VEGF sequence, SEQ ID NO:2, CCACAGTGCA TACGTGGGCT CCAACAGGTC CTCTT, along with additional sequence to facilitate their cloning into the pBI vector, were synthesized by standard oligonucleotide synthesis procedures. These HRE were then used to construct the vectors in Example 5.

### Example 5: Generation of hypoxia-inducible expression vectors

The large size and limited unique cloning sites of currently available adenoviral vectors restrict their usefulness in the multiple subcloning steps required for the construction of the HYPR and HYPRA viruses. Therefore, the construction and testing of hypoxia/HIF-dependent promoters and the subsequent subcloning of the E1 and anti-angiogenic angiostatin gene was performed using modified pBI and pBI-GL mammalian expression vectors (Clontech, Palo Alto, CA, FIG. 2). The resulting gene cassette within the modified pBI plasmid is subsequently used in the construction of the recombinant HYPR and HYPRA adenoviruses.

The pBI vector can be used to express two genes of interest from a bidirectional tetracycline (tet) responsive promoter which contains 7 copies of the tet-responsive elements flanked by two minimal CMV. The pBI-GL vector, contains the Luciferase and LacZ/β-Gal reporter genes under the regulation of the same. The pBI and pBI-GL mammalian expression vectors where modified such that the tet-responsive elements were replaced with hypoxia/HIF-responsive elements (HRE). Xho1 recognition sites were introduced into the pBI and pBI-GL plasmids 5' and 3' of the tet-responsive element by site directed mutagenesis (QuikChange Site-Directed Mutagenesis Kit, Stratagene, La Jolla, CA). The tet-response element was then removed by digestion with Xho 1. Oligonucleotides that span the HRE within the 5' flanking region of the VEGF gene and the 3' flanking region of the EPO gene were synthesized (see example 4), concatemerized and then cloned in tandem (head to tail orientation) into the Xho1 site of the modified pBI and pBI-GL TET vectors. Constructs designated pBI-HRE and pBI-GL-HRE which contain one to six tandem copies of the VEGF or EPO HRE's in both the 5' and 3' orientations were generated.

### Example 6: Testing of bi-directional hypoxia/HIF-inducible reporter gene expression.

The pBI-GL-HRE constructs were examined for their ability to bidirectionally express the luciferase and β-gal reporter genes in response to hypoxia and/or HIF. In addition, the influence of copy number and orientation on the basal activity and hypoxia specific induction of the reporter genes was also examined. Finally, the basal activity and induction capability of the VEGF versus EPO HRE's were compared in order to determine response element that confer optimal regulation in glioma cell lines.

Initially the reporter gene activity ofthe 24 pBIGL-HRE constructs in the LN-229 glioma cell line under normoxic vs. hypoxic conditions were examined. The constructs were transiently transfected into the LN-229 cells using GenePortertransfectionreagent (Gene Therapy Systems, San Diego, CA). The cells were allowed to recover from the transfection procedure overnight and were then incubated under normoxic (20.8% O₂) or hypoxic (1% O₂) conditions for 48 h. The original pBI and pBI-GL plasmids were used as negative controls for these experiments. Luciferase and β-gal enzymatic activities were measured using a commercial assay (Tropic Dual-Light chemiluminescent reporter gene assay system, Applied Biosystems, Foster City, CA). Enzymatic activities were then normalized to total protein in the cellular extracts using a modified Bradford protein assay (Bio-Rad, Hercules, CA).

Based on the data obtained in LN-229 cells, 8 of the 24 constructs (pBI-GL VEGF-5L, 6L, 3R, 4R, 5R, and 6R and pBI-GL EPO-3L and 6L) were selected for further analysis. These 8 constructs displayed the lowest levels of reporter gene activity under normoxic conditions and the highest bi-directional induction of reporter gene activity in response to hypoxia. To confirm and extend the bi-directional reporter gene activity data obtained in LN-229 cells, these 8 constructs were also tested in two other glioma cell lines (U251MG, U138MG) under normoxia and hypoxia (1% O₂) (FIG. 3).

These results demonstrated that the VEGF and EPO HRE induce bi-directional gene expression under hypoxic conditions in the plasmids. Importantly, high induction was exhibited when the tandem copies were facing both in the 5' or 3' direction (L versus R in the construct name), suggesting that the bi-directional induction of the reporter genes was not dependent upon the orientation of the tandem copies. Finally, the background expression of these constructs under normoxic conditions was minimal and not significantly greater than that seen with the pBI-GL vector.

### Example 7: Hypoxia and/or HIF-responsive viruses

Two commonly used methods to generate recombinant adenoviruses involve either homologous recombination in mammalian cells or direct ligation of DNA fragments in the adenoviral genome. Recently, a new system, referred to as pAdEasy (Stratagene, La Jolla, CA), (He, T.C. et al., [1998] Proc. Natl. Acad. Sci. 95:2509-14) has allowed for the recombination procedures to be performed in bacteria. In the pAdEasy system a gene of interest is first cloned into a shuttle plasmid whose polylinker is flanked by adenoviral sequences. These flanking adenoviral sequences allow homologous recombination with an adenoviral plasmid which contains all of the adenoviral genome except for the E1 and E3 viral regions. The recombination product is then transfected into the 293 packaging cell line (ATCC, Rockville, MD) to generate recombinant adenovirus. In order to establish the pAdEasy system within the context of this invention, a replication-deficient adenovirus which expresses the green fluorescent protein (GFP) was generated and GFP expression was verified in infected LN-229 brain tumor cells.

### Example 8: Generation of replication-competent recombination adenovirus HYPR-Ad with the pAdEasy system

Example 8 describes the generation of conditional attenuated adenoviruses that selectively replicate under conditions of hypoxia and/or HIF activation by construction of a recombinant adenovirus with the adenoviral E1A gene under an exogenous hypoxia/HIF-dependent promoter (HRE coupled to minimal CMV promoters).

Adenoviruses arc DNA viruses that infect both dividing and quiescent cells. Re-entry of infected quiescent cells into the cell cycle is required for viral DNA replication and ultimately, viral progeny production. The expression of E1A gene products is essential for these viral functions and adenoviruses which lack the E1A gene region are replication-deficient. The adenoviral E1A gene is the first transcription unit to be expressed from a constitutively active promoter region. Products of the E1A gene exhibit a wide range of biological activities including the modulation of cellular and viral transcription (including the induction of E1B gene transcription) and the induction of DNA synthesis in quiescent cells. However, deregulation of cell growth control by E1A induces apoptosis through p53 dependent and independent mechanisms and ultimately interferes with viral progeny production. The prevention of apoptosis during wild type adenovirus infection is mediated by expression of the adenoviral E1B gene products. The E1B gene encodes two proteins, 21K and 55K, which function independently to inhibit E1A-induced apoptosis. The E1B 21K protein is homologous in sequence, and function to the Bcl-2 family of apoptosis regulators and blocks E1A induced apoptosis as well as many other apoptotic stimuli. The infection of cells with adenoviruses lacking E1B 21K function leads to the appearance of extensive nuclear and viral DNA degradation (deg phenotype) and enhanced cytopathie effect (cyt phenotype). The E1B 55K, in conjunction with the adenoviral E4-orf6 gene product, has two functions during viral production: to directly interact with and inactivate p53, and later in viral production to facilitate the transport of viral late mRNA while inhibiting the transport of most cellular mRNA.

Adenoviruses which lack E1B 55K function, such as ONYX-015, are capable of efficient viral progeny production in many human cells independently of the functional status of p53. However, these mutant viruses exhibit drastically reduced viral yield in a subset of human cell lines. It is currently believed that this mutant phenotype results primarily from the absence of the late mRNA transport function of E1B 55K and that some cell lines may have compensatory pathways that the mutant virus can utilize. Based on the important role ofthe E1B gene products during viral progeny production, HYPR-Ad's as well as an Ad-CMV-E1 may be generated that contain this gene. This may be accomplished by introducing into an E1 deleted adenoviral vector, a DNA cassette containing the adenoviral E1 genomic region (E1A, E1B, and IX genes) in which the E1A gene is regulated by an exogenous hypoxia-inducible promoter (FIG. 6).

The genomic E1 region from nucleotides 501 to 4105 of adenovirus type 5 (encompassing the E1A gene region and the E1B and IX transcription units) were amplified by Pfu Turbo DNA polymerase (Stratagene) using DNA extracted from the d1309 virus (which is wild type for the E 1 gene region but has a substitution in the E3 gene region making it safer for laboratory use, was obtained from Dr. E.Harlow, Massachusetts General Hospital, Boston, MA). The resulting 3.6 Kb amplified DNA product was cloned downstream of an hypoxia/HIF-inducible promoter containing 6 VEGF HRE coupled to a minimal CMV promoter (derived from pBI-VEGF-6R). The HRE-CMV-E1 cassette was subsequently subcloned in the adenoviral shuttle vector (pShuttle) (FIG. 5). Recombinants were selected for kanamycin resistance and recombination was confirmed by multiple restriction endonuclease analyses. In the final phase, the recombinant adenoviral plasmids were linearized with Pac1 to expose the inverted terminal repeats and then transfected using lipofectamine reagent (Gibco-BRL, Gaithersburg, MD) into the 293 packaging cell line. The resulting virus (HYPR-Ad1) was harvested using standard protocols and characterized as described below. For comparative purposes we also generated an adenovirus in which the E1 region is regulated by a constitutively active CMV promoter (Ad-CMV-E1).

### Example 9: Expression of recombinant viral gene products in transfected cells under hypoxic and normoxic conditions

The E1A and E1B viral proteins are constitutively expressed in the human 293 cells as a result of the stable integration of these genes in the 293 cellular genome. Therefore, 293 cells cannot be used to confirm expression of these viral proteins. To confirm expression of these proteins from the recombinant adenoviruses, U251MG and LN-229 cells were infected with the Ad-CMV-E1 and HYPR-Ad1. Protein expression was examined by Western blotting of infected cells using monoclonal anti-Adenovirus Type 5 E1A, E1B 55Kd and 21Kd antibodies. Uninfected cells served as negative control (FIG. 7A-B). E1A is activated by hypoxia in HYPR-Ad1. E1A is known to activate the expression of other viral promoters including early E1B gene regulation. This explains the increased expression of E1B gene products under hypoxia. Late E1B gene regulation involves other factors and may explain the increased expression of E1B21K under normoxia at 2-3 days post-infection.

These experiments demonstrate that the recombinant adenoviruses were able to express constitutively (Ad-CMV-E1) or conditionally (HYPR-Ad1) E1A and E1B gene products. This demonstrates that the hypoxia-dependent regulation seen in the transient reporter gene assays is maintained in the context of the adenovirus genome.

### Example 10: Cytolysis of tumor cells in a hypoxia dependent manner

The ability of HYPR-Ad1 and Ad-CMV-E1 to induce cytolysis in the LN-229 glioma cell line was determined by infection with increasing volumes of the two viruses (FIGS. 8A-D). One set of cells was incubated under normoxic conditions (20.8% O₂) and the other set of cells was incubated under hypoxic conditions (1% O₂). As a negative control, uninfected LN229 gliomas cells were used. The cells were examined daily for evidence of cytopathic effect (CPE). The use of subsaturating viral concentrations for infection enabled us to follow the progression of CPE over time. These preliminary data show that after 6 days of normoxia (not shown) or hypoxia (FIG. 8A) mock infected LN-229 cells are confluent. A small number of cells round-up and appear as dark spots surrounded by a white halo. Some of these cells arc dividing and some detach from the monolayer, a typical behavior for this cell line once it is at confluence. Cells infected with Ad-CMV-E1 (FIG. 8B) show clear signs of CPE, many cells round-up and detach from the monolayer. The morphology of cells infected with HYPR-Ad1 and maintained under normoxia is similar to that of uninfected cells, with perhaps a slight increase in round-up cells (FIG. 8C). In contrast, most cells infected with HYPR-Ad1 and maintained under hypoxia (FIG. 8D) underwent cytolysis similar to Ad-CMV-E1 infected cells. These results suggest that HYPR-Ad1 is conditionally replication-competent since it induces CPE under hypoxic conditions.

### Example 11: Delivery of recombinant virus to brain tumors

The experiments described in this section involves intracerebral injection of tumor cells and adenovirus into nude mice by stereotactic technique. 9L rat glioma tumor cells (5x10⁴ cells, 5 µl volume) were stereotactically implanted in the brain of syngeneic Fisher 344 rats. Eleven days later, a replication-deficient adenovirus expressing LacZ (1.8x10¹⁰ particles, 24 µl volume) was co-injected with inert colloidal carbon particles (0.5 µl) into the tumor using the same stereotactical co-ordinates as for tumor cell injection. The rat was sacrificed 24 h after virus injection and the brain extracted and analyzed by serial sectioning. The section was processed to detect β-gal expression and cells expressing the virally encoded enzyme are visible in the blue area. Black grains arc colloidal carbon particles deposited along the needle track. This section revealed that 60-80% of the tumor cells along the needle tract were infected and express the LacZ protein. See FIG. 9 for results.

### Example 12: Hypoxia-inducible alkaline phosphate expression

A plasmid construct comprised of an alkaline phosphatase gene under the control of an hypoxia-inducible promoter was transfected into LN229 glioma cells. Clones were placed into individual wells of a 48-well platc and exposed to either normoxic or hypoxic conditions. Clones that assayed positive for expression of alkaline phosphatase under hypoxic conditions and negative under normoxic conditions were retained and examined further. Clones that tested positive under both normoxic and hypoxic conditions were discarded.

### Example 13: Identification of compounds that modulate the hypoxia-inducible pathway

Equal numbers of cells derived from a clone of LN229 glioma cells transformed with an expressible plasmid construct comprising an hypoxia/HIF-responsive promoter operably linked to the gene for alkaline phosphatase are added to the wells of a 96-well microtiter plate. Cells are incubated overnight at 37°C to allow them to adhere. The microtiter plates and cells containing the reporter construct are simultaneously equilibrated to a desired O₂ concentration, *i.e.,* hypoxic or normoxic, or treated with a compound known to induce transcription of genes driven by the hypoxia/HIF-responsive promoter.

An appropriate amount oftest compound diluted into assay buffer is then placed into each well. During initial screens each well has a different test compound except for the control well which have either no compound or an inactive compound. After a selected period of time the reactions are assayed for alkaline phosphatase activity. The alkaline phosphatase activity can be detected visually or spectrophotometrically. Compounds which reduce or increase the level of expression of alkaline phosphatase under conditions which induce hypoxia-responsive genes are classified as modulators of the hypoxia/HIF-inducible pathway.

Compounds that test positive in the initial screening assay are further examined by performing the same assay as described above, except for varying the concentration of the test compound to determine the concentration of compound that inhibits 50% of the level of expression of the alkaline phosphatase activity (IC₅₀). Compounds that have an IC₅₀ in these assays of less than 500 µm are preferred; compounds that have an IC₅₀ of less than 100 µm are more preferred; and those that have an IC₅₀ of less than 10 µm are highly preferred.

### Example 14: In vivo evaluation of compounds modulating expression of hypoxia-responsive genes.

Experimental tumors are grown in immunocompromised mice by placing cells derived from a clone of LN229 glioma cells transformed with an expressible plasmid construct comprising an hypoxia-responsive promoter operably linked to the gene for alkaline phosphatase. When tumors have reached a particular size, a compound which has been shown to modulate induction of hypoxia-responsive genes is administered to the mice. An inactive compound, or no compound, is administered to other mice in the experimental group as a negative control.

After a determined time, the experimental tumors are removed from the mice, and sections of these tumors are assayed for expression of alkaline phosphatase. Control tumors will show pockets of alkaline phosphatase expression, but the majority of the cells in the tumor will now show alkaline phosphatase expression. Expression of alkaline phosphatase in an experimental tumor treated with a compound which modulates expression of hypoxia-responsive genes by higher percentage of cells than is seen in the control tumor confirms that the compound is capable of reaching tumor cells and is active *in vivo* to stimulate hypoxia-responsive gene transcription. However, alkaline phosphatase expression by a lower percentage of cells than is seen in the control tumor confirms that the compound is capable of reaching tumor cells and is active *in vivo* to inhibit hypoxia-responsive gene transcription.

### Example 15: Reduction of the growth of xenografted glioma cells in immunocompromised mice.

LN229 cells were implanted subcutaneously into the left flank of nu/nu mice. When the average tumor volume [volume = (length x width²)/2) reached 75 mm³ (arrow), the mice were divided into three groups and 0.66 x 10⁸ pfu of adenovirus (HYPR-Ad1 or d1309) or PBS (vehicle) was injected daily for five days. Foroty-nine days following the injection protocol the mice were sacrificed (due to the large size of the PBS injected tumors) and the tumors were harvested. One of the vehicle mice died immediately following the completion of the injection protocol due to an unknown cause and one of the d1309 mice was sacrificed on day 57 due to excessive weight loss related to an eye infection. We found no difference in the growth ofLN229 tumors that were injected with PBS versus not injected (data not shown).

At the time of harvest the average size of the HYPR-Ad1 injected tumors (circles) were 5.3 times smaller than PBS (squares) injected tumors. The d1309 injected tumors (diamonds) were 34 times smaller than PBS injected tumors. The d13-9 injected tumors were 6.4 times smaller than HYPR-Ad1 injected tumors.

## Claims

1. A recombinant virus comprising one to six copies of a hypoxia and/or HIF (hypoxia-inducible factor) responsive element to make a bidirectional hypoxia and/or HIF responsive element, said element linked to a minimal promoter at both ends, wherein one minimal promoter controls the expression of a gene which modulates replication of the virus, wherein said virus selectively replicates in and causes cytolysis of hypoxic tissues and cells or cells and tissues containing an active HIF pathway.

2. The recombinant virus of claim 1, wherein the virus is an adenovirus, herpes virus, herpes-like virus, retrovirus or picornavirus.

3. The recombinant virus of claim 2, wherein the virus is an adenovirus.

4. The recombinant virus of claim 3, wherein said gene which modulates replication of the virus is the EIA gene.

5. The recombinant virus of claim 4, wherein said virus is adenovirus type 5.

6. The recombinant virus of claim 1, further comprising a gene encoding a protein having anti-angiogenic activity, wherein said gene is operably linked to a minimal promoter whose expression is controlled by the bidirectional hypoxia and/or HIF responsive element.

7. The recombinant virus of claim 6, wherein the gene encodes angiostatin, thrombospondin-1, thrombospondin-2, endostatin, PF4, BAI1, IL4, ADAMTS, PEDF, or fragments thereof.

8. The recombinant virus of claim 1, wherein the hypoxia responsive element induces expression of at least one gene below normoxia.

9. The recombinant virus of claim 1, wherein the cells or tissues are tumor cells or tissues.

10. The recombinant virus of claim 1, wherein said promoter is CMV minimal promoter.

## Patentansprüche

1. Rekombinantes Virus, umfassend ein bis sechs Kopien eines Hypoxie- und/oder HIF- (Hypoxie induzierbarer Faktor) -responsiven Elements, um ein bidirektionales Hypoxie- und/oder HIF-responsives Element herzustellen, wobei das Element an beiden Enden mit einem Minimalpromotor verbunden ist, wobei ein Minimalpromotor die Expression eines Gens, das die Replikation des Virus moduliert, kontrolliert, wobei das Virus selektiv in hypoxischen Geweben und Zellen, oder Zellen und Geweben, die einen aktiven HIF-Weg enthalten, repliziert und deren Zytolyse verursacht.

2. Rekombinantes Virus nach Anspruch 1, wobei das Virus ein Adenovirus, Herpesvirus, herpesähnliches Virus, Retrovirus oder Picornavirus ist.

3. Rekombinantes Virus nach Anspruch 2, wobei das Virus ein Adenovirus ist.

4. Rekombinantes Virus nach Anspruch 3, wobei das Gen, das die Replikation des Virus moduliert, das EIA-Gen ist.

5. Rekombinantes Virus nach Anspruch 4, wobei das Virus ein Typ 5 Adenovirus ist.

6. Rekombinantes Virus nach Anspruch 1, weiter ein Gen umfassend, das ein Protein kodiert, das anti-angiogene Aktivität aufweist, wobei das Gen funktionell mit einem Minimalpromotor verbunden ist, dessen Expression von dem bidirektionalen Hypoxie- und/oder HIF-responsiven Element kontrolliert wird.

7. Rekombinantes Virus nach Anspruch 6, wobei das Gen Angiostatin, Thrombospondin-1, Thrombospondin-2, Endostatin, PF4, BAl1, IL4, ADAMTS, PEDF oder Fragmente davon, kodiert.

8. Rekombinantes Virus nach Anspruch 1, wobei das Hypoxie-responsive Element bei unternormaler Sauerstoffversorgung die Expression von mindestens einem Gen induziert.

9. Rekombinantes Virus nach Anspruch 1, wobei die Zellen oder Gewebe TumorZellen oder -gewebe sind.

10. Rekombinantes Virus nach Anspruch 1, wobei der Promotor ein CMV Minimalpromotor ist.

## Revendications

1. Virus recombinant comprenant une à six copies d'un élément sensible à l'hypoxie et/ou à un HIF (facteur inductible par l'hypoxie) pour fabriquer un élément bidirectionnel sensible à l'hypoxie et/ou à un HIF, ledit élément étant lié à un promoteur minimal aux deux extrémités, dans lequel un promoteur minimal contrôle l'expression d'un gène qui module la réplication du virus, où ledit virus se réplique sélectivement dans des cellules et des tissus hypoxiques ou des cellules et des tissus contenant une voie de HIF active, et provoque la cytolyse de ces cellules et tissus.

2. Virus recombinant selon la revendication 1, où le virus est un adénovirus, le virus herpétique, un virus de type herpétique, un rétrovirus ou un picornavirus.

3. Virus recombinant selon la revendication 2, où le virus est un adénovirus.

4. Virus recombinant selon la revendication 3, où ledit gène qui module la réplication du virus est le gène EIA.

5. Virus recombinant selon la revendication 4, où ledit virus est un adénovirus de type 5.

6. Virus recombinant selon la revendication 1, comprenant en outre un gène codant pour une protéine ayant une activité anti-angiogénique, où ledit gène est lié de manière fonctionnelle à un promoteur minimal dont l'expression est contrôlée par l'élément bidirectionnel sensible à l'hypoxie et/ou à un HIF.

7. Virus recombinant selon la revendication 6, dans lequel le gène code pour l'angiostatine, la thrombospondine-1, la thrombospondine-2, l'endostatine, PF4, BAl1, IL4, ADAMTS, PEDF, ou leurs fragments.

8. Virus recombinant selon la revendication 1, dans lequel l'élément sensible à l'hypoxie induit l'expression d'au moins un gène en dessous de la normoxie.

9. Virus recombinant selon la revendication 1, dans lequel les cellules ou les tissus sont des cellules ou des tissus tumoraux.

10. Virus recombinant selon la revendication 1, dans lequel ledit promoteur est un promoteur minimal de CMV.
